**Europäisches Patentamt**

**European Patent Office**

**Office européen des brevets**

(19)

(11) Veröffentlichungsnummer : **0 116 280**
**B1**

(12) **EUROPÄISCHE PATENTSCHRIFT**

(45) Veröffentlichungstag der Patentschrift :
29.07.87

(51) Int. Cl.⁴ : **A 61 N 1/05, A 61 B 5/04**

(21) Anmeldenummer : **84100158.9**

(22) Anmeldetag : **09.01.84**

(54) **Bipolare Elektrode für medizinische Anwendungen.**

(30) Priorität : **11.01.83 DE 3300694**

(43) Veröffentlichungstag der Anmeldung :
**22.08.84 Patentblatt 84/34**

(45) Bekanntmachung des Hinweises auf die Patenterteilung : **29.07.87 Patentblatt 87/31**

(84) Benannte Vertragsstaaten :
**DE FR IT NL**

(56) Entgegenhaltungen :
**EP-A- 0 054 781**
**DE-A- 2 165 622**
**DE-A- 2 613 072**
**DE-A- 2 842 318**
**DE-A- 2 922 354**
**DE-A- 3 046 732**
**US-A- 4 281 669**
**BIOMEDIZINISCHE TECHNIK, Band 25, Nr. 7, 8. Juli/August 1980, Berlin, H.J. BISPING "Neue Schrittmachersonden - ein Bericht aus Montreal", Seiten 170-175**

(73) Patentinhaber : **Siemens Aktiengesellschaft Berlin und München**
**Wittelsbacherplatz 2**
**D-8000 München 2 (DE)**

(72) Erfinder : **Botvidsson, Lars**
**Trollvaegen 7**
**S-175 70 Jaerfaella (SE)**
Erfinder : **Mund, Konrad, Dr.**
**Langenbrucker Weg 6**
**D-8521 Uttenreuth (DE)**

## Beschreibung

Die Erfindung betrifft eine bipolare Elektrode für medizinische Anwendungen, insbesondere eine implantierbare Herzschrittmacherelektrode, mit einem isolierten Leitersystem, mindestens einer aktiven und einer in Abstand dazu entlang des Leitersystems angeordneten indifferenten Elektrode.

Bei unipolarer Herzschrittmacherbehandlung entstehen oft Probleme mit Muskelstimulierungen und/oder Muskelinhibierungen an dem Gehäuse des Herzschrittmachers, das üblicherweise die indifferente Elektrode in dem Elektrodensystem darstellt. Eine mögliche Lösung dieses Problems besteht bekannterweise darin, ein bipolares Elektrodensystem auszunutzen, d. h. die indifferente Elektrode in der Nähe der aktiven Elektrode(n) noch im Inneren des Herzens anzuordnen. Aufgrund des geringeren Abstandes zwischen aktiver und indifferenter Elektrode ist man bestrebt, die Impedanz des Elektrodensystemes zu senken. Bekannte indifferente Elektroden bestehen beispielsweise aus einem zylindrischen Körper aus einer Platin/Iridium-Legierung mit einer Oberfläche von ca. 50 mm². Aufgrund der relativ niedrigen Doppelschichtkapazität von Platin/Iridium (10 $\mu$F/cm², 1 kHz) muss diese Elektrode eine so grosse Oberfläche haben, um die Polarisationsverluste in vertretbaren Grenzen zu halten.

Aufgrund der grossen Dimensionen der indifferenten Elektrode ergeben sich jedoch erhebliche mechanische Probleme. Denkt man sich beispielsweise einen isolierten elektrischen Leiter mit 3 mm Durchmesser, so muss der zylindrische Körper der indifferenten Elektrode etwa 5 mm lang sein, um die geforderte Oberfläche zu besitzen. Das ergibt eine erhebliche Versteifung des ansonsten extrem flexiblen elektrischen Leiters in der Nähe der aktiven Elektrode. Handelt es sich beispielsweise um eine Herzkammerelektrode, die in der Spitze der linken Herzkammer appliziert werden soll, so liegt die indifferente Elektrode ebenfalls in dieser Herzkammer. Bei der grossen Anzahl von Verbiegungen, der dieser elektrische Leiter ausgesetzt ist, stellt eine derartig kräftige Versteifung eine grosse Belastung dar, die das Risiko, dass es zu einer Beschädigung der Isolierung oder zu einem Bruch des Leiters in der Nähe der Versteifung kommt, erhöht.

Der vorliegenden Erfindung liegt die Aufgabe zugrunde, die Fläche der indifferenten Elektrode bei unveränderten oder sogar noch verminderten Polarisationsverlusten zu reduzieren und damit die mechanischen Beanspruchungen der Elektrode in diesem Bereich erheblich zu senken.

Diese Aufgabe wird erfindungsgemäss dadurch gelöst, dass die Länge der indifferenten Elektrode in Richtung des Leitersystems so kurz bemessen ist, daß die Flexibilität dieses Leitersystems in diesem Bereich nicht wesentlich gestört wird, und dass zumindest der aktive Bereich der indifferenten Elektrode eine Oberflächenschicht aufweist, die an der Phasengrenze zur Körperflüssigkeit eine hohe Doppelschichtkapazität besitzt. Diese hohe Doppelschichtkapazität sorgt für eine geringe elektrochemische Impedanz und ergibt nur einen geringen Polarisationsanstieg während der Reizimpulse. Diese Verminderung, die man bei den Polarisationsverlusten erhält, kann dazu ausgenutzt werden, die Fläche der indifferenten Elektrode zu reduzieren und damit die mechanischen Beanspruchungen der Elektrode zu vermindern.

Vorteilhafterweise kann die indifferente Elektrode dazu eine aufgerauhte Oberfläche besitzen oder insgesamt aus einem porösen Material, beispielsweise aus einer gesinterten Metall-Legierung, bestehen. Ebenso ist es möglich, die indifferente Elektrode auf der Oberfläche mit einer Schicht aus aktiviertem Glaskohlenstoff zu versehen, die eine extrem hohe Doppelschichtkapazität von bis zu 0,1 F/cm² aufweist.

Eine besonders vorteilhafte indifferente Elektrode erhält man dadurch, dass die Oberflächenschicht aus einer porösen Schicht aus einem Carbid, Nitrid oder Carbonitrid wenigstens eines der Metalle Titan, Vanadium, Zirkonium, Niob, Molybdän, Hafnium, Tantal oder Wolfram aufweist. Die porösen Carbid-, Nitrid- oder Carbonitridschichten befinden sich auf einem elektrisch leitenden Trägermaterial, beispielsweise Platin, Titan oder einer Metall-Legierung wie Elgiloy. Die damit erzielbaren Doppelschichtkapazitäten und dadurch bedingten möglichen Flächenreduzierungen liegen etwa in der gleichen Grössenordnung wie bei Glaskohlenstoffschichten. Herstellungstechnisch sind diese Schichten jedoch vorzuziehen.

Um Mischpotentiale zu vermeiden, ist in Weiterbildung der Erfindung vorgesehen, dass sich zwischen dem Trägermaterial und der porösen Schicht eine dichte Schicht aus einem entsprechenden Material wie die poröse Schicht befindet.

Die Doppelschichtkapazitäten der erfindungsgemässen indifferenten Elektroden liegen etwa um einen Faktor 10 bis 100 über denen bekannter Elektroden, so dass eine wesentliche Verringerung der Oberfläche der indifferenten Elektrode möglich ist. Diese Elektrode schrumpft praktisch auf einen schmalen Ring zusammen, der die mechanischen Eigenschaften des Leitersystems nun praktisch kaum noch verändert, d. h. die hohe Elastizität der Elektrodenleitung ist auch im Bereich der indifferenten Elektrode weitgehend gewährleistet.

Weiterhin lässt sich mit Hilfe der erfindungsgemässen indifferenten Elektrode, je nachdem, wie gross die Reduktion der Fläche gewählt wird, neben den verbesserten mechanischen Eigenschaften auch noch die elektrischchemische Impedanz des gesamten Elektrodensystemes senken, wodurch die Empfindlichkeit des Systemes weiter gesteigert wird.

Die dünnen porösen Nitrid- Carbid- oder Carbonitrid-Schichten werden vorzugsweise durch reaktives Jonenplattieren, d. h. durch physikalische Dampfabscheidung auf dem als Substrat dienenden Trägermaterial wie Titan oder Platin aufgebracht. Durch Dampfdruckänderungen lassen sich hierbei auch in einem kontinuierlichen Fertigungsprozess zunächst dichte Schichten und anschliessend poröse Schichten aus dem gleichen Material abscheiden.

## Patentansprüche

1. Bipolare Elektrode für medizinische Anwendungen, insbesondere eine implantierbare Herzschrittmacherelektrode, mit einem isolierten Leitersystem, mindestens einer aktiven und einer in Abstand dazu entlang des Leitersystems angeordneten indifferenten Elektrode, dadurch gekennzeichnet, dass die Länge der indifferenten Elektrode in Richtung des Leitersystems so kurz bemessen ist, dass die Flexibilität. dieses Leitersystems in diesem Bereich nicht wesentlich gestört wird, und dass zumindest der aktive Bereich dieser indifferenten Elektrode eine Oberflächenschicht aufweist, die an der Phasengrenze zur Körperflüssigkeit eine hohe Doppelschichtkapazität besitzt.

2. Bipolare Elektrode nach Anspruch 1, dadurch gekennzeichnet, dass die Oberflächenschicht durch eine aufgerauhte Oberfläche der indifferenten Elektrode gebildet ist.

3. Bipolare Elektrode nach Anspruch 1, dadurch gekennzeichnet, dass die indifferente Elektrode zumindest im Oberflächenbereich aus gesintertem Material mit hoher Porösität und grosser innerer Oberfläche besteht.

4. Bipolare Elektrode nach Anspruch 1, dadurch gekennzeichnet, dass die Oberflächenschicht aus Aktivkohle, insbesondere aktiviertem Glaskohlenstoff, besteht.

5. Bipolare Elektrode nach Anspruch 1, dadurch gekennzeichnet, dass die Oberflächenschicht aus einer porösen Schicht aus einem Carbid, Nitrid oder Carbonitrid wenigstens eines der Metalle Titan, Vanadium, Zirkonium, Niob, Molybdän, Hafnium, Tantal oder Wolfram besteht.

6. Bipolare Elektrode nach einem der Ansprüche 1, 2, 4 oder 5, dadurch gekennzeichnet, dass die Oberflächenschicht eine Schichtdicke zwischen 1 und 100 μm, vorzugsweise zwischen 5 und 20 μm, aufweist.

7. Bipolare Elektrode nach Anspruch 5 oder 6, dadurch gekennzeichnet, dass sich zwischen dem Trägermaterial und der porösen Schicht eine dichte Schicht aus einem entsprechenden Material wie die poröse Schicht befindet.

8. Bipolare Elektrode nach Anspruch 7, dadurch gekennzeichnet, dass die dichte Schicht eine Schichtdicke zwischen 2 und 10 μm aufweist.

## Claims

1. A bipolar electrode for medicinal applications, particularly, but not exclusively, an implantable heart pacemaker electrode, comprising an insulated conductor system, at least one active electrode and one inert electrode arranged at a distance therefrom along the conductor system, characterised in that the length of the inert electrode in the direction of the conductor system is sufficiently short to prevent the flexibility of the conductor system from being substantially disturbed in this region ; and that at least the active zone of this inert electrode is provided with a surface layer which has a high double layer capacitance at the phase boundary to the body fluid.

2. A bipolar electrode as claimed in Claim 1, characterised in that the surface layer is formed by a roughened surface of the inert electrode.

3. A bipolar electrode as claimed in Claim 1, characterised in that at least in the surface region, the inert electrode is made of sintered material which has a high level of porosity and a large internal surface.

4. A bipolar electrode as claimed in Claim 1, characterised in that the surface layer consists of activated carbon, in particular, activated vitreous carbon.

5. A bipolar electrode as claimed in Claim 1, characterised in that the surface layer consists of a porous layer made of a carbide, nitride or carbonitride of at least one of the metals titanium, vanadium, zirconium, niobium, molybdenum, hafnium, tantalum, or tungsten.

6. A bipolar electrode as claimed in one of Claims 1, 2, 4 or 5, characterised in that the surface layer has a thickness of between 1 and 100 μm, preferably between 5 and 20 μm.

7. A bipolar electrode as claimed in Claim 5 or 6, characterised in that a dense layer consisting of a material corresponding to that of the porous layer is arranged between the carrier material and the porous layer.

8. A bipolar electrode as claimed in Claim 7, characterised in that the dense layer has a thickness of between 2 and 10 μm.

## Revendications

1. Electrode bipolaire pour applications médicales, notamment électrode de stimulateur cardiaque qui est susceptible d'être implantée et qui comprend un système conducteur isolé, au moins une électrode active, et au moins une électrode indifférente disposée à distance de celle-ci le long du système conducteur, caractérisée en ce que la longueur de l'électrode indifférente, suivant la direction du système conducteur, est si petite qu'il n'est sensiblement pas porté atteinte à la souplesse de ce système conducteur dans cette région, et en ce qu'au moins la région active de cette électrode indifférente comporte une couche superficielle qui possède, à la limite des phases avec le liquide de l'organisme, une grande capacité à doubles couches.

2. Electrode bipolaire suivant la revendication 1, caractérisée en ce que la couche superficielle est formée par une surface rugueuse de l'électrode indifférente.

3. Electrode bipolaire suivant la revendication 1, caractérisée en ce que l'électrode indifférente est constituée, au moins dans la région superficielle, d'un matériau fritté d'une grande porosité et d'une grande surface spécifique.

4. Electrode bipolaire suivant la revendication 1, caractérisée en ce que la couche superficielle est en charbon actif, notamment en produit obtenu par décomposition thermique de composés aromatiques avec réticulation.

5. Electrode bipolaire suivant la revendication 1, caractérisée en ce que la couche superficielle est en une couche poreuse de carbure, nitrure ou carbonitrure d'au moins l'un des métaux que sont le titane, le vanadium, le zirconium, le niobium, le molybdène, l'hafnium, le tantale ou le tungstène.

6. Electrode bipolaire suivant l'une des revendications 1, 2, 4 ou 5, caractérisée en ce que la couche superficielle a une épaisseur comprise entre 1 et 5 microns et, de préférence, comprise entre 5 et 20 microns.

7. Electrode bipolaire suivant la revendication 5 ou 6, caractérisée en ce qu'entre le matériau support et la couche poreuse est interposée une couche dense en un matériau correspondant à celui de la couche poreuse.

8. Electrode bipolaire suivant la revendication 7, caractérisée en ce que la couche dense a une épaisseur comprise entre 2 et 10 microns.